# EUROPEAN PATENT SPECIFICATION

(11) **EP 1 159 984 B1**
(45) Date of publication and mention of the grant of the patent: **28.07.2004**
(21) Application number: 01108396.1
(22) Date of filing: 03.04.2001
(51) Int. Cl.: A61M 25/10, A61B 17/12

(54) **Occlusion catheter for the ascending aorta**
Okklusionskatheter für der Aufsteigenden Aorta
Catheter d'occlusion pour l' aorte ascendante

(30) Priority: 29.05.2000 JP 2000158152
(43) Date of publication of application: 05.12.2001
(73) Proprietor: Kabushiki Kaisha Vayu, Nagoya-shi, Aichi-ken (JP)
(72) Inventor: Yozu, Ryohei, Yokohama-shi, Kanagawa-ken (JP); Tsutsui, Nobumasa, Nagoya-shi, Aichi-ken (JP); Kumeno, Takashi, Ama-gun, Aichi-ken (JP)
(74) Representative: Leson, Thomas Johannes Alois, Dipl.-Ing.

(56) References cited:
- WO-A-98/48884
- WO-A-99/30766
- US-A- 4 950 239
- US-A- 5 021 045
- US-A- 6 021 340
- US-A- 6 045 531

## Description

### BACKGROUND OF THE INVENTION

### i) Field of the Invention

The present invention relates to an occlusion catheter for the ascending aorta according to the preamble of claim 1.

### ii) Prior Art

When a cardiac surgery is performed, the blood flow in the ascending aorta is obstructed generally using a conventional cross-clamp through an opening in a patient's chest formed by thoracotomy.

Recently, there have been attempts to obstruct the blood flow in the ascending aorta using an occlusion catheter for the ascending aorta (hereinafter referred to as "an occlusion catheter") inserted through a femoral artery, for example, without performing an open chest operation.

An occlusion catheter of this type comprises a tube and a balloon disposed on the circumference of the distal end of the tube. The tube is inserted through the femoral artery and advanced through the thoracic aorta to the ascending aorta, in which the distal end of the tube is placed and the balloon is inflated, with the result that the blood flow is obstructed.

In another occlusion catheter, a cardiac muscle protective drug supplied from the proximal end of the tube is delivered to the distal end of the tube and released from a drug release aperture provided at the distal end of the tube.

These conventional occlusion catheters, however, have the following problems: The conventional occlusion catheter to be inserted through the femoral artery may hinder the blood flow directed to the lower limb, particularly, of a patient whose blood vessel in the lower limb is thin. In such a case an occlusion catheter of this type cannot be used.

The conventional occlusion catheter requires a guide wire in order to push the occlusion catheter throughout a long path from the femoral artery to the ascending aorta. As a result, a lumen to pass the guide wire therethrough must be provided in the tube, which leads to a relatively large diameter of the catheter and therefore a further factor of hindrance to the blood flow.

Furthermore, since the conventional occlusion catheter is long enough to extend from the femoral artery to the ascending aorta, the flow path resistance of the lumen in the tube is so large that the flow rate of the cardiac muscle protective drug cannot be easily increased. Although the flow rate of the cardiac muscle protective drug can be increased by simply having a lumen of a larger inner diameter, such a lumen necessarily requires a catheter of a larger diameter, which may hinder the blood flow toward the lower limb.

When inserted through the femoral artery, the conventional occlusion catheter may have its balloon damaged in the case where the blood vessel in the lower limb or the aorta is calcified, or cannot be easily advanced through a meandering blood vessel.

While the tube preferably is flexible enough to curve to a certain extent for better operation in the blood vessel, the tube having an excessive flexibility cannot support the balloon which is pushed by the blood flow from a pump oxygenator or the infusion pressure of the cardiac muscle protective drug. In this case, the balloon together with the tube is to be displaced from the proper indwelling position. To avoid such displacement of the balloon, a substantially hard tube is generally employed and operationality in the blood vessel is necessarily sacrificed.

The conventional occlusion catheter has a structure wherein displacement of the catheter tube in the axial direction is easily conducted to the balloon. Specifically, when the proximal end of the catheter tube is displaced in the axial direction, the distal end of the catheter tube is also displaced in the axial direction, with the result that the balloon is displaced as well. Thus, it is not easy to retain the balloon in the proper indwelling position.

WO 98/48884 discloses a generic occlusion catheter for the ascending aorta, comprising a catheter tube having at least a first lumen and a second lumen independent of each other; and a balloon provided on the outer circumference of the distal end of said catheter tube for being inflated/deflated in accordance with supply or drainage of a fluid through said first lumen in order to obstruct the blood flow within the ascending aorta when inflated, said catheter tube being provided with an aperture for releasing a drug supplied through said second lumen in the position closer to the proximal end than the site where the blood flow is obstructed by said balloon on the outer circumference of the distal end of said catheter tube.

US-A-5 021 045 and US-A-4 950 239 each disclose an occlusion catheter having a balloon formed of polyurethane.

It is the object of the present invention, to provide a occlusion catheter for the ascending aorta having an improved position retainability of the catheter.

The object is solved by the occlusion catheter for the ascending aorta having the features of claim 1.

The invention is further developed as it is defined in the dependent claims.

### BRIEF DESCRIPTION OF THE DRAWINGS

Preferred embodiments of the present invention will be described hereinafter with reference to the drawings, in which:
FIG. 1 is a side view of an occlusion catheter according to a first embodiment of the invention;
FIG. 2 is a cross-sectional view of the occlusion catheter shown in FIG. 1 taken along line II-II;
FIGS. 3A through 3C are schematic views showing modifications of the positions for forming drug release apertures;
FIGS. 4A through 4D are exemplary views illustrating how to use the occlusion catheter;
FIG. 5 is a schematic diagram showing the operational state of the above occlusion catheter;
FIG. 6 is a side view of an occlusion catheter according to a second embodiment of the invention;
FIG. 7A is a longitudinal sectional view in the vicinity of the balloon of the occlusion catheter shown in FIG. 6;
FIG. 7B is a sectional view of the tip end of a catheter tube;
FIGS. 8A through 8C show how concavities provided in the balloon are transformed;
FIG. 9 is a schematic diagram showing the operational state of the occlusion catheter according to the second embodiment;
FIG. 10A is a schematic view of an occlusion catheter reinforced with a linear metal wire;
FIG. 10B is a schematic view of an occlusion catheter reinforced with a spiral reinforcing member;
FIGS. 11A through 11D are cross-sectional views showing modified reinforcement members.

### DETAILED DESCRIPTION OF THE PREFERRED EMBODIMENTS

### First Embodiment

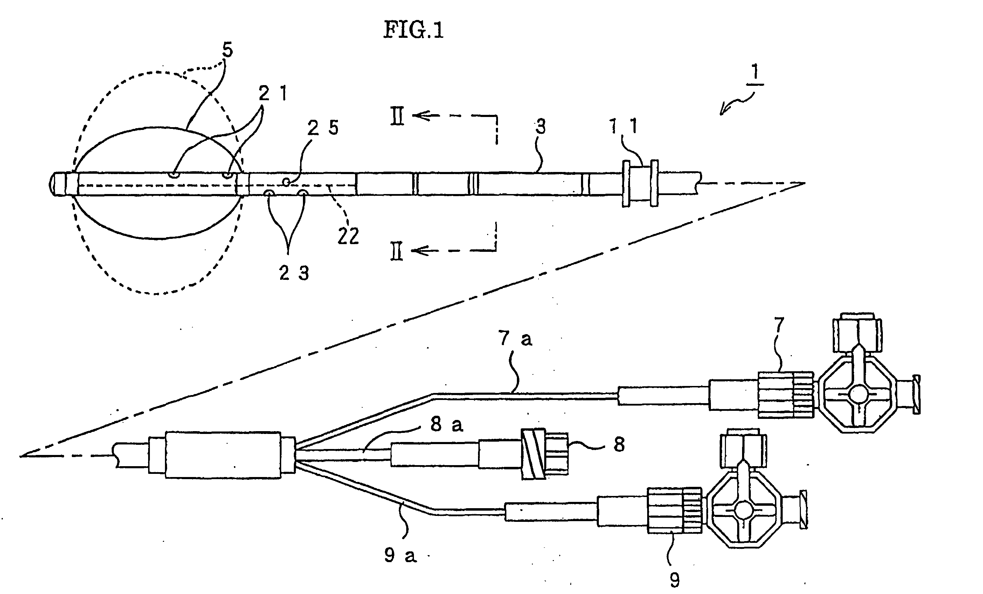

As shown in Fig. 1, an occlusion catheter for the ascending aorta 1 according to the present invention is provided with a catheter tube 3, a balloon 5, a first connector 7, a second connector 8, a third connector 9 and a tie cushion 11, etc.

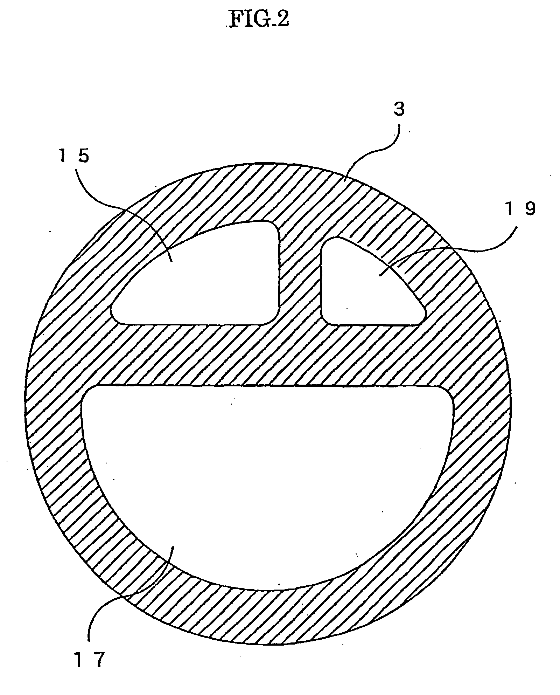

The catheter tube 3 made of polyurethane is long enough to include a portion with a length of 400mm and an outer diameter of 3.6mm which is insertable into a blood vessel. As shown in FIG. 2, a first lumen 15, a second lumen 17 and a third lumen 19 are formed separately from one another within the catheter tube 3.

The first lumen 15 has one end communicating with the first connector 7 through a first auxiliary tube 7a and the other end communicating with a plurality of inflation agent supply and drainage apertures 21 having a diameter of 1mm and formed inside the balloon 5. The first lumen 15 is used as a path for guiding an inflation agent (e.g. a physiological salt solution) supplied from the first connector 7 side to the inside of the balloon 5. At least one inflation agent supply and drainage aperture 21 is preferably arranged in a region adjoining a joint of the balloon 5 and the catheter tube 3. It is because when an operation of filling the balloon 5 with the inflation agent to remove air bubbles before insertion of the catheter is performed, air bubbles can easily be removed through the above supply and drainage aperture 21 arranged in the region adjoining the above joint. It is also preferable that the circumference of the supply and drainage aperture 21 is colored (black, for example) to improve visibility and facilitate guidance of air bubbles to the supply and drainage aperture 21.

The second lumen 17 has one end communicating with the second connector 8 through a second auxiliary tube 8a and the other end communicating with drug release apertures 23 each aperture having a diameter of 1.5mm and being formed in the side wall of the catheter tube 3 closer to the proximal end than the balloon 5. The second lumen 17 is used as a passage for guiding a drug (e.g. a cardiac muscle protective drug) injected from the second connector 8 side to the drug release aperture 23.

The third lumen 19 has one end communicating with the third connector 9 through a third auxiliary tube 9a and the other end communicating with a blood pressure measuring aperture 25 having a diameter of 0.8mm and formed in the side wall of the catheter tube 3 closer to the proximal end than the balloon 5. The third lumen 19 is used for measuring the blood pressure outside the blood pressure measuring aperture 25 with a pressure sensor (not shown) connected to the third connector 9 via a liquid (blood, a physiological salt solution, or the like) which is introduced into the third lumen 19.

The balloon 5 is made of polyurethane and has a length of 30mm, an outer diameter of 20mm ( the molded dimension) and a film thickness of 75 µm. The outer diameter can be increased further to such an extent that the balloon 5 occludes the ascending aorta, as indicated by a dotted line in FIG. 1.

The tie cushion 11 is a hollow cylindrical member which facilitates secure fixation of a Nelaton's tube (not shown) to the catheter tube 3 when tying them with a thread and prevents crash of the tube 3 by the external force at the same time.

It is preferable to house a metal wire 22 in the catheter tube 3 in order to improve stability when the tube 3 is placed in an indwelling position. Detailed description of the metal wire will be presented in connection with a second embodiment of the present invention.

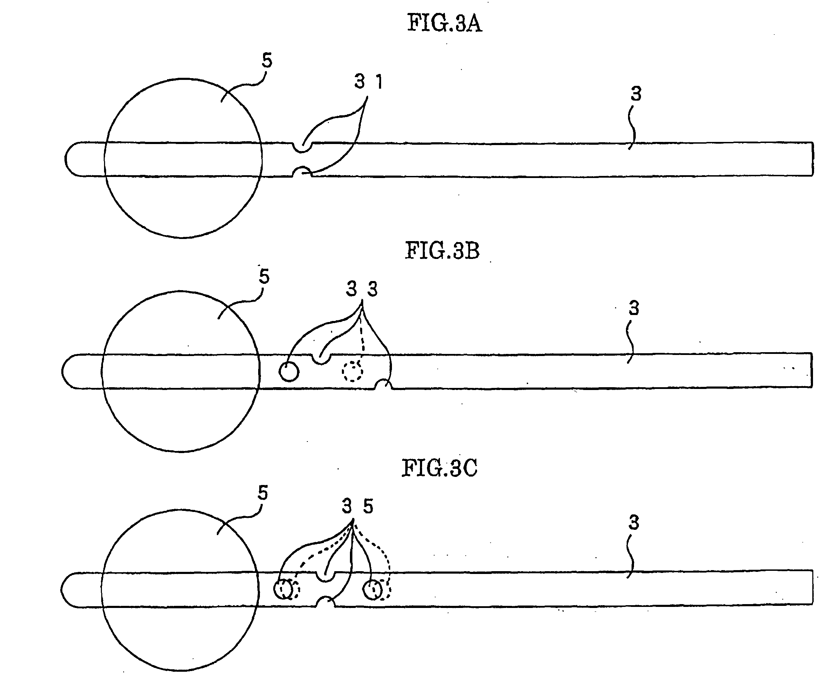

While the two drug release apertures 23 are formed in the same direction in the above catheter tube 3, it is possible to form two drug release apertures 31 in two different directions as shown in FIG. 3A. In this case, even if one of the drug release apertures 31 is shut tight by a blood vessel wall, the drug can be surely released from the other drug release aperture 31.

To avoid the possibility of decreasing the strength of the catheter tube 3 around the region where the drug release apertures 31 are formed in the same axial position of the catheter tube 3, a plurality of drug release apertures 33 are preferably formed in different axial positions of the catheter tube 3, as shown in FIG. 3B. In the case where four drug release apertures 33 are arranged spirally as shown in FIG. 3B, these apertures are not in the same axial position of the catheter tube 3 and the drug are released in four different directions.

Releasing the drug in four different directions does not always require the drug release apertures to be arranged spirally and allows drug release apertures 35, for example, to be arranged as shown in FIG. 3C.

An example of how to use the occlusion catheter 1 will now be described.

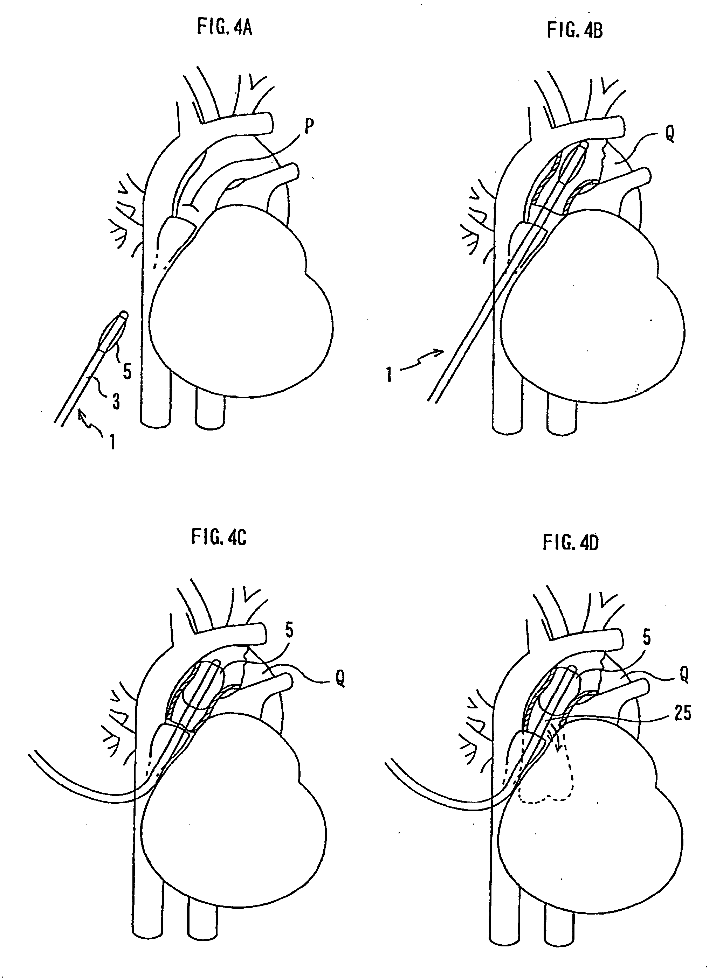

Firstly, the chest of a patient is incised to expose the ascending aorta and the ascending aorta is perforated at an insertion site P thereon (FIG. 4A). The usual perforation at the insertion site P includes puncture using a needle to provide a hole and expansion of the hole with a dilator, but other methods may be employed.

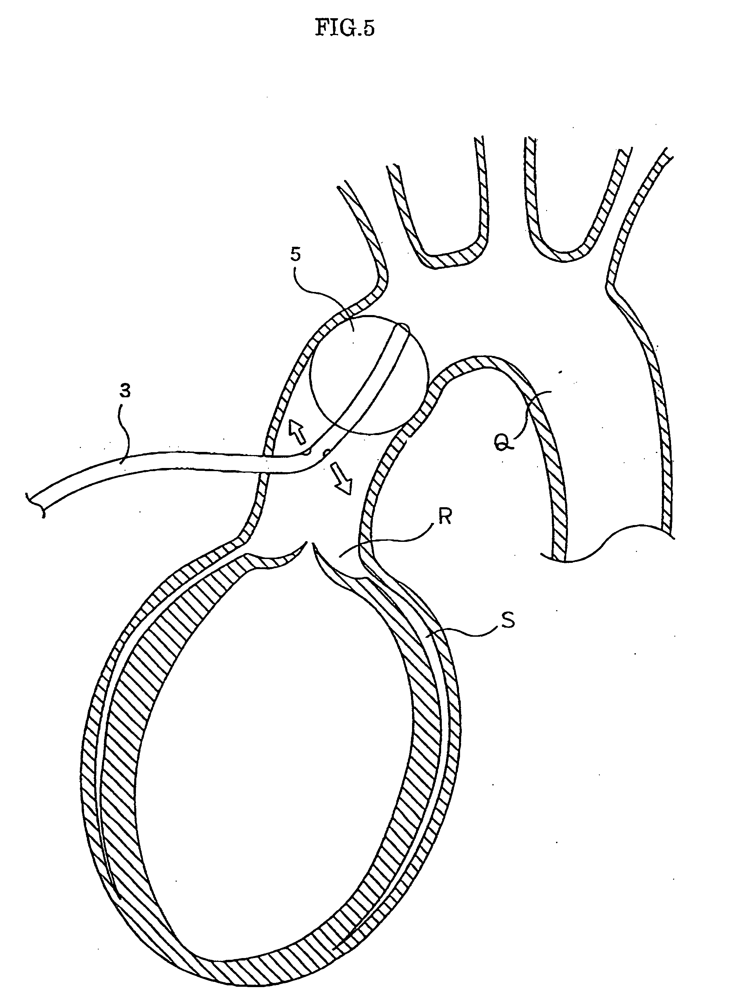

Subsequently, the above occlusion catheter 1 is inserted into the ascending aorta Q (FIG. 4B) and then fixed by ligating the surrounding of the insertion site with a suture thread by cigarette suture technique. The suture thread used for ligation is passed through, for example, the Nelaton's tube and fixed to the Nelaton's tube in a position distant from the insertion site using a mosquito clamp or the like in order to prevent the thread from loosening in the ligated part. The Nelaton's tube in turn is fixed to the catheter tube 3 by tying the same with a thread within the region of the tie cushion 11. The balloon 5 is placed in the proper indwelling position while checking the position by X-ray fluoroscopy, and then is inflated (FIG. 4C). As a result, the ascending aorta is occluded by the balloon 5, as shown in FIG. 5. The obstruction of the blood flow is confirmed by angiography, and if necessary, a cardiac muscle protective drug is released from the drug release aperture 25 (FIG. 4D) to be delivered to the vicinity of the coronary ostium R and therefore to the coronary artery S.

As described above, use of the occlusion catheter 1 permits a surgeon when performing a cardiac surgery to insert the occlusion catheter 1 directly into the ascending aorta in the vicinity of the heart after making an incision in the chest so that the blood flow can be obstructed as well as the cardiac muscle protective drug (indicated by the arrows in FIG. 5) can be delivered to the vicinity of the coronary ostium R by supplying the cardiac muscle protective drug through the second lumen 17.

Thus, even when a cross-clamp is not or cannot be used, it is unnecessary to insert an occlusion catheter through a femoral artery, with the result that hindrance of the blood flow toward the lower limb is prevented, that the flow rate of the drug such as a cardiac muscle protective drug can be easily increased, and that a calcified or meandering blood vessel of the lower limb does not become an obstacle upon insertion of the catheter.

### Second Embodiment

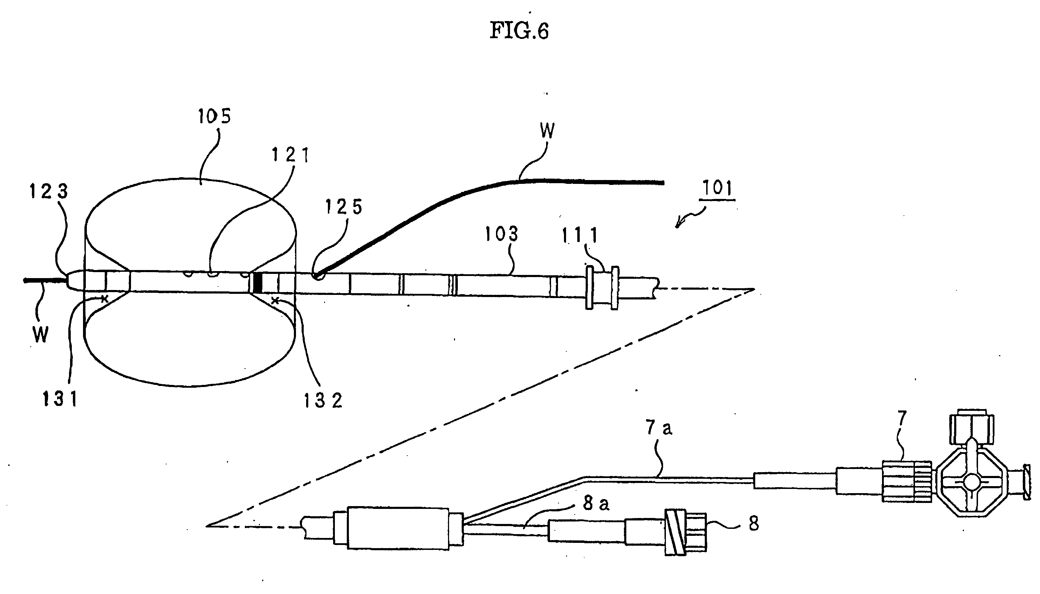

As shown in Fig. 6, an occlusion catheter 101 according to a second embodiment of the present invention is provided with a catheter tube 103, a balloon 105, a first connector 7, a second connector 8, a third connector 9 and a tie cushion 111, etc.

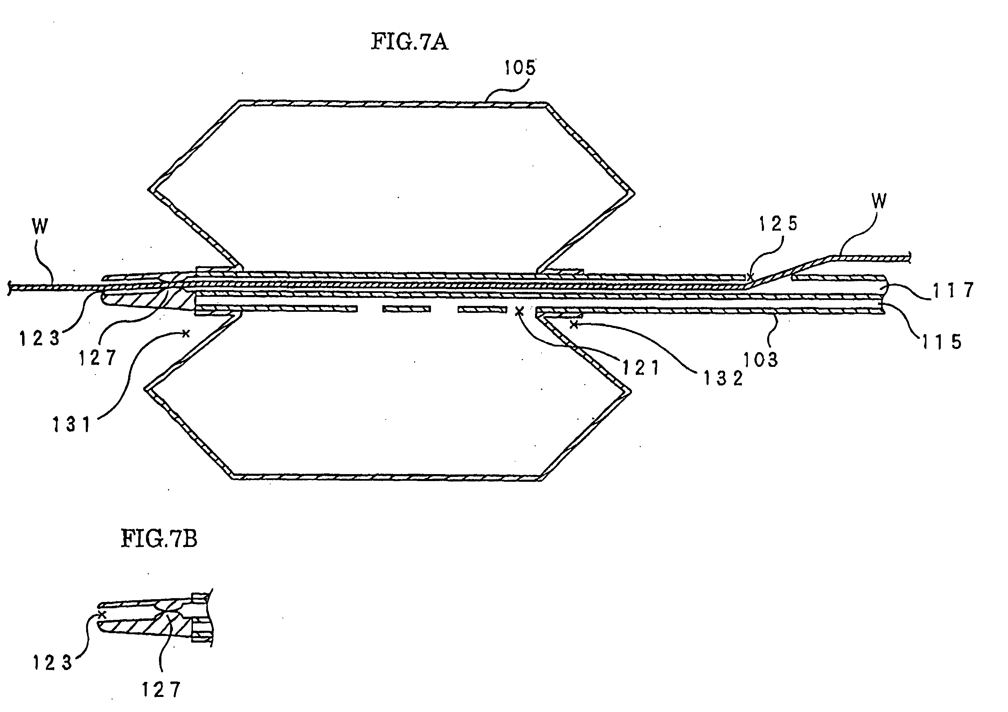

The catheter tube 103 made of polyurethane is long, and a first lumen 115 and a second lumen 117 are formed therein separately from each other as shown in FIG. 7A.

The first lumen 115 has one end communicating with the first connector 7 through a first auxiliary tube 7a (see FIG. 6) and the other end communicating with a plurality of inflation agent supply and drainage apertures 121 formed inside the balloon 105. The first lumen 115 is used as a passage for guiding an inflation agent (e.g. a physiological salt solution) supplied from the first connector 7 side to the inside of the balloon 105.

The second lumen 117 has one end communicating with the second connector 8 through a second auxiliary tube 8a, the other end communicating with a distal end opening 123 formed at the distal end of the catheter tube 103, and a side aperture 125 communicating the second lumen 117 and the outside of the catheter tube 103, the side aperture 125 being arranged closer to the proximal end than the balloon 105. One portion of the second lumen 117 from the distal end opening 123 to the side aperture 125 is used for passing a guide wire W therethrough. The other portion of the second lumen 117 from the side aperture 125 to the second connector 8 is used for guiding a drug (e.g. a cardiac muscle protective drug) injected from the second connector 8 side to the side aperture 125, and for measuring the blood pressure outside the side aperture 125 with a pressure sensor (not shown) connected to the second connector 8.

The second lumen 117 is provided with a valve 127 therein, which is made of an elastic body having rubber elasticity (e.g. silicone rubber in the present embodiment). The valve 127 has an insertion hole to pass the guide wire W therethrough, the insertion hole being shut tight to prevent the fluid from flowing through at least when the guide wire is not inserted into the insertion hole, as shown in 7B. Thus, when the drug is injected from the second connector 8 side into the second lumen 117, the drug is all released from the side aperture 125 without escaping from the distal end opening 123. When the guide wire W is pressed against the insertion hole, the valve 127 is elastically deformed by the force from the guide wire W and the guide wire W is allowed to pass through the insertion hole expanded due to the elastic deformation.

The balloon 105 is a bag made of a polyurethane thin film (e.g. having a film thickness of 75 µm in the present embodiment) and has a size large enough to occlude the ascending aorta once inflated. The balloon 105 made of polyurethane has an advantage that when inflated, a desired configuration can be obtained more easily than a balloon made of a rubber material. The balloon 105 has at its both ends, concavities 131 and 132 depressed toward the inside of the balloon 105, and therefore has an apple-like shape as a whole. These concavities 131 and 132 are formed by joining both ends of the balloon 105 to the catheter tube 103 in such a manner that the distance between the two joined parts is shorter than the entire length of the balloon 105.

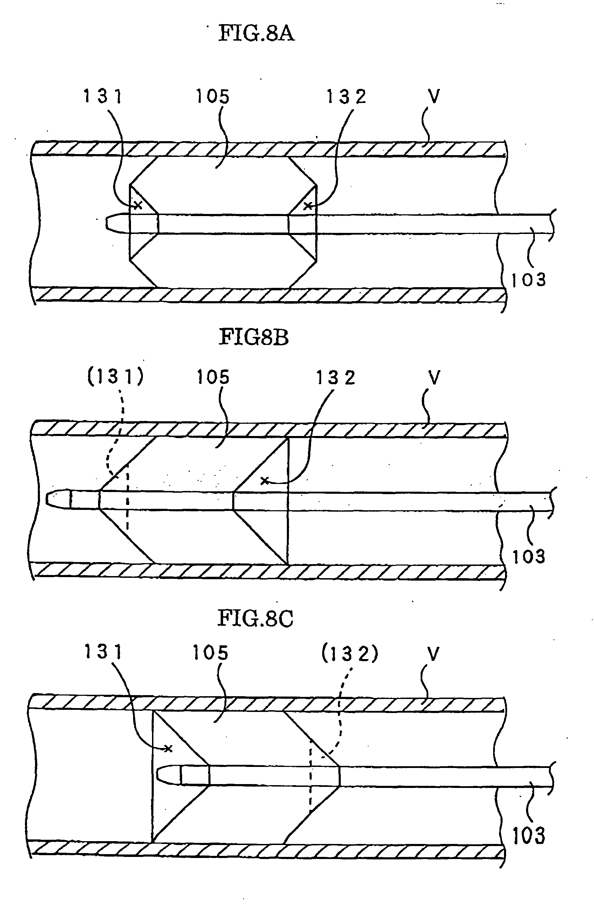

Once the balloon 105 is inflated in the proper indwelling position in the blood vessel V as shown in FIG. 8A and if the catheter tube 103 is displaced for some reason, the concavity 131 becomes convex while the concavity 132 becomes further concave as shown in FIG. 8B. This prevents the balloon 105 from being displaced by following the catheter tube 103. If the catheter tube 103 is displaced in the opposite direction to the above case, the concavity 132 becomes convex while the concavity 131 becomes further concave as shown in FIG. 8C, whereby the balloon catheter 105 is prevented from being displaced by following the catheter tube 103.

The tie cushion 111 is a cylindrical member for preventing the catheter tube 103 from being adversely affected by the force caused when a Nelaton's tube (not shown) is tied and secured to the catheter tube 103 with a thread.

It will now be described how to use the occlusion catheter 101 of the second embodiment.

Firstly, the chest of a patient is incised to expose the ascending aorta and the ascending aorta is perforated to provide a hole in an insertion site thereon. Then, the guide wire W is inserted to secure an insertion route and the hole is expanded with a dilator operated along the guide wire W.

Subsequently, the above occlusion catheter 101 is inserted into the ascending aorta along the guide wire W, and the balloon 105 is placed in the proper indwelling position by locating the balloon 105 using X-ray fluoroscopy. The second lumen 117 in the catheter tube 103 is used as a lumen for guide wire insertion.

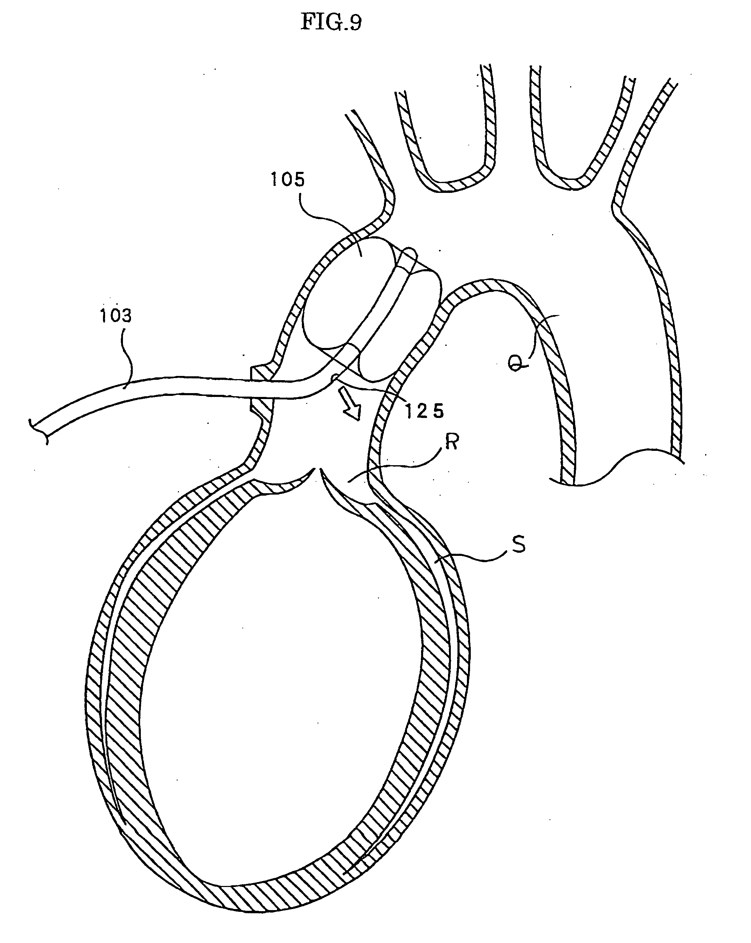

After the guide wire W is pulled out, the balloon 105 is inflated by supplying an inflation agent through the first lumen 115. As a result, the ascending aorta Q is occluded by the balloon 105, as shown in FIG. 9. The obstruction of the blood flow is to be confirmed by angiography.

When required, a cardiac muscle protective drug is supplied through the second lumen 117 to be released from the side aperture 125 (indicated by the arrow in FIG. 9), with the result that the cardiac muscle protective drug is delivered to the vicinity of the coronary ostium R. While the second lumen 117 is used as a drug delivery lumen in this case, the second lumen 117 is also used for measuring the blood pressure outside the side aperture 125 with a pressure connected to the second connector 8.

As described above, the occlusion catheter 101 with a valve 127 allows the second lumen 117 to be used for three purposes, i.e. for guide wire insertion, drug delivery and blood pressure measurement at the same time. By this, the diameter of the catheter tube 103 can be reduced compared with the catheter tube containing independent lumens corresponding to these purposes, respectively, and therefore the catheter tube 103 can be more flexible. Thus, the displacement of the catheter tube 103 at the proximal end does not directly affect the position of the distal end, and specifically the balloon 105 is not displaced from the proper indwelling position because of a slight displacement of the proximal end of the catheter tube 103.

In the occlusion catheter 101, the displacement of the catheter tube 103 also can be absorbed by deforming the concavities 131 and 132 which are provided for the balloon 105. Therefore, even if the displacement of the catheter tube 103 at the proximal end reaches the distal end, the displacement of the catheter tube 103 at the distal end still does not directly reach the center portion of the balloon 105, which is not displaced from the proper indwelling position because of a slight displacement of the proximal end of the catheter tube 103.

Although the present invention has been described by way of examples, the present invention is not limited to the aforementioned embodiments, and can variously be modified.

For example, although the occlusion catheter 101 is provided with the valve 127 in the catheter tube 103 as well as the concavities 131 and 132 in the balloon 105, the balloon 105 need not be provided with the concavities 131 and 132 if the diameter of the catheter tube 103 is reduced enough, by providing the valve 127 for the catheter tube 103, to achieve the advantage of retaining the balloon 105 in the proper indwelling position.

Oppositely, the catheter tube 103 need not be provided with the valve 127 if the balloon 105 is provided with the concavities 131 and 132 to achieve the same advantage of retaining the balloon 105 in the proper indwelling position. It is also possible to provide a balloon catheter without a lumen for guide wire insertion if not required, wherein the balloon catheter is inserted without using a guide wire and the same advantage of retaining the balloon 105 in the proper indwelling position can be achieved by providing the concavities 131 and 132 in the balloon 105.

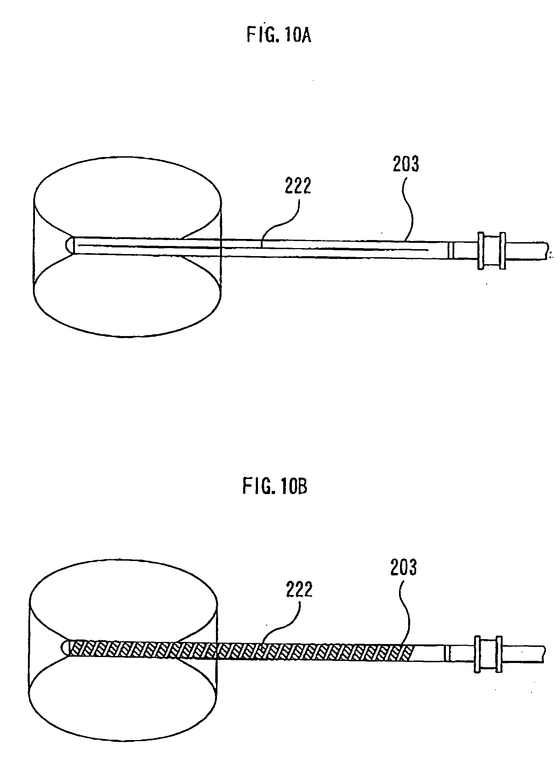

As part of the present invention the catheter tube 203 contains a linear metal wire 222 or a spiral reinforcing member 223 therewithin, as shown in FIG. 10A or FIG. 10B, in order to improve position retainability of the catheter. The material for the metal wire 222 is an elastic material, such as nickel-titanium alloy or stainless steel alloy (e.g. SUS304 according to *JIS*(*Japanese Industrial Standards*)), or an inelastic material, such as stainless steel alloy (SUS316 according to *JIS)* or copper. As the material for the spiral reinforcing member 223, a resin which is harder than the material for the catheter tube 203 may be employed in addition to the above-mentioned materials for the metal wire 222. The metal wire 222 or the spiral reinforcing member 223 has a given length, which preferably ranges from 5cm to 20cm, from the distal end of the catheter tube 203.

With the metal wire 222 contained, the catheter tube may have a configuration having the same outer diameter in the portion from the distal end to the midsection (beyond the part to be fixed with the tie cushion toward the proximal end) and having a decreasing outer diameter, i.e. tapered, from the midsection toward the proximal end. This structure can effectively avoid a kink in the catheter which is anticipated being caused due to a sudden change of flexural strength of the catheter on the borderline between the metal wire and a supporting portion for supporting or fastening the wire.

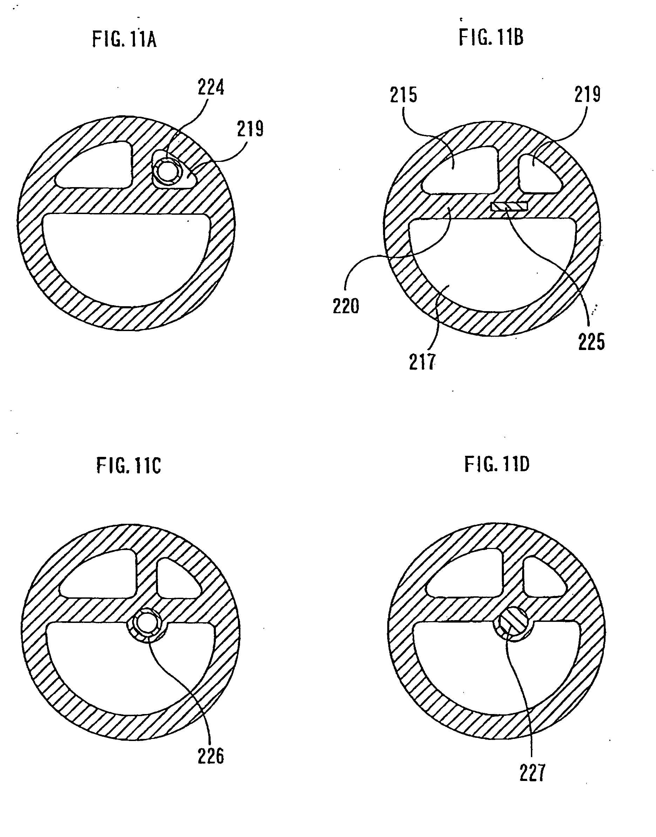

A metal pipe, a flat metal plate or a solid metal cylinder may be employed instead of the linear metal wire 222 or the spiral reinforcing member 223. FIGS. 11A through 11D show these examples. In FIG. 11A, a blood pressure measuring lumen 219 contains a metal pipe 224, which serves to reinforce the front end of the catheter tube and is used for blood pressure measurement with a fluid passing through the pipe. In FIG. 11B, a partition wall 220 separating a first lumen 215 and a third lumen 219 from a second lumen 217 contains a metal plate 225. In FIG. 11C, the partition wall 220 contains a metal pipe 226. In FIG. 11D, the partition wall 220 contains a solid metal cylinder 227.

Although the second lumen 117 in the second embodiment is used for guide wire insertion, drug delivery and blood pressure measurement at the same time by providing the side aperture 125, the second lumen may have a structure without the side aperture 125 if the second lumen 117 is used, for example, for filling a contrast agent therein to obtain images of the catheter tube 103. Even when such a structure is employed, one lumen can be used for both guide wire insertion and contrast agent filling, and therefore the diameter of the catheter tube 103 is reduced to provide an advantage of retaining the balloon 105 in the proper indwelling position.

Although the guide wire W is led out through the side aperture 125 in the second embodiment, the guide wire W may be passed throughout the length of the catheter tube 103. In this case, since it is unnecessary to pass the guide wire W through the side aperture 125, a plurality of openings each opening having a small area may be provided instead of the side aperture 125.

When these plurality of openings are provided in different directions, even if one of the openings is shut by the blood vessel wall, release of the drug is ensured through the rest of the openings. Some of the plurality of openings, of course, may be such that the guide wire W can be inserted thereinto.

Although it has been described that the occlusion catheter 101 is used for obstructing the blood flow in the ascending aorta, the occlusion catheter according to the invention used for obstructing the blood flow in a blood vessel other than the ascending aorta achieves the same advantage that the balloon is retained in the proper indwelling position. Moreover, when the occlusion catheter for ascending aorta according to the present invention is used for widening a narrowed part of a blood vessel, widening effect is further ensured because the balloon can be retained in the proper indwelling position.

Although the occlusion catheter according to each of the above-described embodiments is inserted after opening the chest to expose the ascending aorta, such an opening of the chest is not always necessary. For example, it is possible to make a hole in the chest to provide access to the ascending aorta from the outside, and insert the occlusion catheter into the ascending aorta through the hole.

## Claims

1. An occlusion catheter for the ascending aorta comprising:
a catheter tube (203) having at least a first lumen (215) and a second lumen (217) independent of each other; and
a balloon provided on the outer circumference of the distal end of said catheter tube (203) for being inflated/deflated in accordance with supply or drainage of a fluid through said first lumen (215) in order to obstruct the blood flow within the ascending aorta when inflated,
said catheter tube (203) being provided with an aperture for releasing a drug supplied through said second lumen (217) in the position closer to the proximal end than the site where the blood flow is obstructed by said balloon on the outer circumference of the distal end of said catheter tube (203),
**characterised in that**
said catheter tube (203) includes a portion being reinforced with a reinforcement member (222, 223; 224; 225; 226; 227), and
said reinforcement member (222, 223; 224; 225; 226; 227) is a linear metal wire (222), a spiral reinforcing member (223), a metal pipe (224; 226), a metal plate (225), or a solid metal cylinder (227).

2. The occlusion catheter for the ascending aorta according to claim 1, wherein at least two apertures for each releasing a drug are formed more proximally than said balloon in different directions, respectively.

3. The occlusion catheter for the ascending aorta according to claim 2, wherein said at least two apertures are formed in different positions, respectively, relative to the axial direction of said tube.

4. The occlusion catheter for the ascending aorta according to any one of claims 1 to 3, wherein said portion being reinforced with said reinforcement member (222, 223; 224; 225; 226; 227) extends from the distal end of said catheter tube (203) to a point a predetermined distance away therefrom.

5. The occlusion catheter for the ascending aorta according to any one of claims 1 to 4, wherein said aperture for releasing a drug and the vicinity thereof are reinforced by said reinforcement member (222, 223; 224; 225; 226; 227).

6. The occlusion catheter for the ascending aorta according to any one of claims 1 to 5, wherein said balloon is made of polyurethane.

## Patentansprüche

1. Okklusionskatheter für die Aorta Ascendes, mit:
einer Katheterröhre (203) mit zumindest einem ersten Lumen (215) und einem zweiten Lumen (217), die voneinander unabhängig sind; und
einem Ballon, der an dem Außenumfang von dem distalen Ende der Katheterröhre (203) so vorgesehen ist, dass er gemäß einer Zufuhr oder einem Auslassen eines Fluids durch das erste Lumen (215) aufgeblasen wird/schrumpft, um die Blutströmung innerhalb der Aorta Ascendes zu blockieren, wenn er aufgeblasen ist,
wobei die Katheterröhre (203) mit einer Öffnung zum Auslassen eines durch das zweite Lumen (217) zugeführten Medikamentes an jener Position versehen ist, die näher an dem körpernahen Ende als jene Stelle ist, an der die Blutströmung durch den Ballon an dem Außenumfang des distalen Endes der Katheterröhre (203) blockiert wird,
**dadurch gekennzeichnet, dass**
die Katheterröhre (203) einen Abschnitt aufweist, der durch ein Verstärkungselement (222, 223; 224; 225; 226; 227) verstärkt ist, und
das Verstärkungselement (222, 223; 224; 225; 226; 227) ein gerader Metalldraht (222), ein gewundenes Verstärkungselement (223), ein Metallrohr (224; 226), eine Metallplatte (225) oder ein voller Metallzylinder (227) ist.

2. Okklusionskatheter für die Aorta Ascendes gemäß Anspruch 1, wobei zumindest zwei Öffnungen jeweils zum Auslassen eines Medikamentes noch körpernäher als der Ballon in jeweils unterschiedlichen Richtungen ausgebildet sind.

3. Okklusionskatheter für die Aorta Ascendes gemäß Anspruch 2, wobei zumindest zwei Öffnungen an unterschiedlichen Positionen jeweils bezüglich der axialen Richtung der Röhre ausgebildet sind.

4. Okklusionskatheter für die Aorta Ascendes gemäß einem der Ansprüche 1 bis 3, wobei sich jener Abschnitt, der durch das Verstärkungselement (222, 223; 224; 225; 226; 227) verstärkt ist, von dem distalen Ende der Katheterröhre (203) zu einem Punkt erstreckt, der über einen vorbestimmten Abstand davon entfernt ist.

5. Okklusionskatheter für die Aorta Ascendes gemäß einem der Ansprüche 1 bis 4, wobei die Öffnung zum Auslassen eines Medikaments und deren Nähe durch das Verstärkungselement (222, 223; 224; 225; 226; 227) verstärkt sind.

6. Okklusionskatheter für die Aorta Ascendes gemäß einem der Ansprüche 1 bis 5, wobei der Ballon aus Polyurethan besteht.

## Revendications

1. Cathéter d'occlusion pour l'aorte ascendante comprenant :
un tube cathéter (203) ayant au moins une première lumière (215) et une seconde lumière (217) indépendantes l'une de l'autre; et
un ballonnet prévu sur la circonférence extérieure de l'extrémité distale dudit tube cathéter (203) et destiné à être gonflé/dégonflé selon l'apport ou le drainage d'un fluide à travers ladite première lumière (215) afin de faire obstruction à l'écoulement sanguin à l'intérieur de l'aorte ascendante lorsqu'il est gonflé,
ledit tube cathéter (203) étant muni d'une ouverture afin de libérer un médicament amené par ladite seconde lumière (217) dans la position plus proche de l'extrémité proximale que le site au niveau duquel l'écoulement sanguin est arrêté par ledit ballonnet sur la circonférence extérieure de l'extrémité distale dudit tube cathéter (203),
**caractérisé en ce que** ledit tube cathéter (203) comprend une partie qui est renforcée à l'aide d'un élément de renforcement (222, 223 ; 224; 225 ; 226 ; 227), et
ledit élément de renforcement (222, 223 ; 224 ; 225 ; 226 ; 227) est un fil métallique linéaire (222), un élément de renforcement en spirale (223), un tuyau métallique (224 ; 226), une plaque métallique (225) ou un cylindre métallique plein (227).

2. Cathéter d'occlusion pour l'aorte ascendante selon la revendication 1, dans lequel au moins deux ouvertures destinées à libérer chacune un médicament sont formées de manière plus proximale que ledit ballonnet dans différentes directions, respectivement.

3. Cathéter d'occlusion pour l'aorte ascendante selon la revendication 2, dans lequel lesdites au moins deux ouvertures sont formées dans différentes positions, respectivement, par rapport à la direction axiale dudit tube.

4. Cathéter d'occlusion pour l'aorte ascendante selon l'une quelconque des revendications 1 à 3, dans lequel ladite partie qui est renforcée à l'aide dudit élément de renforcement (222, 223 ; 224 ; 225 ; 226; 227) s'étend depuis l'extrémité distale dudit tube cathéter (203) jusqu'à un point qui en est éloigné d'une distance prédéterminée.

5. Cathéter d'occlusion pour l'aorte ascendante selon l'une quelconque des revendications 1 à 4, dans lequel ladite ouverture destinée à libérer un médicament et ses abords sont renforcés par ledit élément de renforcement (222, 223 ; 224 ; 225 ; 226 ; 227).

6. Cathéter d'occlusion pour l'aorte ascendante selon l'une quelconque des revendications 1 à 5, dans lequel ledit ballonnet est fabriqué en polyuréthane.
